# EUROPEAN PATENT APPLICATION

(11) **EP 1 439 167 A1**
(43) Date of publication of application: **21.07.2004**
(21) Application number: 04000496.2
(22) Date of filing: 13.01.2004
(51) Int. Cl.: C07C 253/34, C07C 255/08

(54) **Recycle of condensed quench overheads in a process for purifying acrylonitrile**

(30) Priority: 14.01.2003 US 439975 P
(71) Applicant: Solutia Inc., St. Louis, Missouri 63141 (US)
(72) Inventor: Monical, Valerie, S., Houston, Texas 77062 (US); Murphy, Richard, D., Alvin, Texas 77511 (US)
(74) Representative: Albrecht, Thomas, Dr.

(57) **Abstract**

An overhead stream from the quench system in a process for purifying acrylonitrile is condensed and recycled directly to the quench system as part of the quench liquid. The stream can be condensed in a column or in a partial condenser.

## Description

### BACKGROUND OF THE INVENTION

This invention relates to the recovery and purification of acrylonitrile made by catalytic ammoxidation of propylene. More particularly, the invention relates to an improvement in the quencher used to process reactor effluent.

In commercial processes for preparation of acrylonitrile from propylene, ammonia, and oxygen (air), the reactor effluent contains, in addition to the desired acrylonitrile product, considerable amounts of by-product hydrogen cyanide, acetonitrile, and other impurities such as succinonitrile and other nitriles. The exact composition of the effluent and the by-products and impurities it contains may vary considerably depending on the ammoxidation reaction conditions and catalyst.

Processes for treating reactor effluents of the type described to separate and recover acrylonitrile product and desired by-products such as hydrogen cyanide and acetonitrile are known. For example, see U.S. Pat. Nos. 3,399,120; 3,433,822; 3,936,360; 4,059,492; 4,166,008; 4,404,064; and 5,895,822, the disclosures of which are incorporated herein by reference. Typically, these processes include introducing the reactor effluent into a quench chamber where it is contacted with water (usually containing sulfuric acid to neutralize excess ammonia from the reaction) to cool the effluent and remove some contaminates such as polymers produced in the reactor. Cooled effluent gases from the quench flow to an absorber column where they are contacted with water. The liquid stream from the bottom of the absorber column contains most of the nitriles produced in the reaction and impurities and is sent to an extractive distillation column. The major portion of the acrylonitrile from the extractive distillation column is obtained in the overhead (distillate) from the column while water and impurities constitute the bottom stream from the column. In accordance with practices of the art, the bottom stream is frequently fed to a secondary distillation or stripper column to separate acetonitrile and water in an overhead stream while the secondary column bottoms containing water and various impurities are recycled to the quench column. It was apparently believed that the impurities in the recycle stream were acceptable in the quench system (see, for example, U.S. Pat. No. 3,960,360).

The large quantity of quench liquid required by the quench column and waste management considerations make the appropriate use of recycle water an important process consideration. Accordingly, improvements in recycle practices are sought by those skilled in the art.

### SUMMARY OF THE INVENTION

The present invention provides an improved process for the production of acrylonitrile. In particular, the present invention provides an improved process for processing the effluent from the reactor. In a preferred embodiment, the overhead stream from the quencher is condensed and a portion of the condensate is recycled back to the quencher with the remainder of the condensate being processed in a distillation column. The overhead stream can be condensed by various procedures including a column with a pump-around loop and a partial condenser. The temperature of the condensation process is determined by the relative economics of available streams for cooling.

### BRIEF DESCRIPTION OF THE DRAWINGS

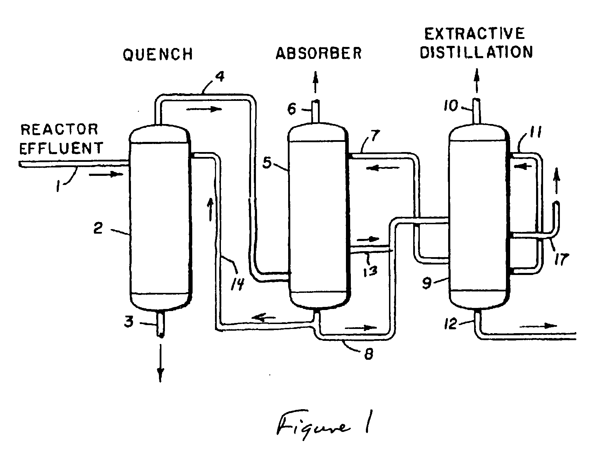

The drawing is a schematic representation of the present invention in the form of a simplified process flow diagram. For simplicity, various recycle streams and heat supply/recovery means which will generally be used in conjunction with the process are not shown.

### DESCRIPTION OF ILLUSTRATIVE EMBODIMENTS

The present invention can advantageously be used for processing of reactor product effluents from reactors in which acrylonitrile is produced by the catalytic ammoxidation of propylene. The commercial production of acrylonitrile by such reactions is well known. The product effluent of such reactions normally contains, in addition to acrylonitrile, by-product hydrogen cyanide, acetonitrile, acrolein, addition compounds of hydrogen cyanide and high boiling and resinous organic compounds.

The reactor product effluents are generally at a temperature of about 870°F as they leave the reactor. They are often quenched in a hot quench system where an aqueous stream is used to provide adiabatic cooling. The source of the material used for the quench and the composition of the stream have a significant impact on the capital cost of the installation, the waste streams produced by the plant, the plant operating costs, and the tendency of the quench to form tars which can significantly impact the quench operability.

Quench liquid can be provided by feeding only "clean" water to the system. For example, fresh treated water or condensate can be utilized. Alternatively, clean water can be used to dilute highboiler rich recycle streams from a downstream stripper. However, such added water as well as water produced in the overall process must eventually be purged and intolerable or expensive waste disposal problems are likely when total process water is unduly increased. Consequently, the derivation of quench liquid from process recycle streams to the extent possible is normally desired.

The present invention is an improvement over existing systems and comprises supplying water to the quench that is derived by condensing liquid from the quench overhead vapors and recycling it to the quench. This makeup water can be condensed in various processes including a column, such as an absorber with a pump-around loop, or in a partial condenser.

In one preferred embodiment, the water is condensed in an absorber column with a pump-around loop with the bottom liquid temperature about 144 °F and the vapor exiting the pump-around section at about 100 °F. A portion of the condensed material is recycled as quench makeup water. The amount of recycle is determined by the required material balance in the quencher. In a preferred embodiment, about one-third of the condensed fluid is recycled. The amount can vary depending upon quencher conditions, including the temperature at which the condensate was generated. The amount of condensate that is recycled can vary from about 15% to 100%. This water can be combined with other recycle streams or with fresh water if necessary to supply the quench system.

The invention can be better understood by reference to the attached drawing, which illustrates a simplified flow diagram of a preferred embodiment of the present invention.

In the present invention, as in conventional practice, the ammoxidation reactor effluent (which may be pre-cooled if desired) is passed through a conduit 1 into a quench column 2 where it is contacted with quench liquid introduced through line 14. (Although this drawing shows a quench column, the quench system may, alternatively, be any gas-liquid contact means such as, for example, Venturi towers, spray towers or the like.) This quench liquid is primarily water and is shown as being obtained as a recycle stream as hereinafter described. However, the quench liquid may be obtained in whole or part from other sources, not shown, if desired. As explained above, recycle quench liquid from process sources may be supplemented or replaced with "clean" water. In addition, sufficient sulfuric acid may be added to neutralize any excess ammonia in the reactor effluent. To minimize formation of highboilers in the quench system itself, the system is preferably operated at as low a temperature and pH as practicable, commensurate with other process considerations.

A bottoms stream containing water and, usually, high concentrations of organic impurities and sulfates exits the quench column through conduit 3 for disposal or further treatment while cooled reactor effluent gas exits through conduit 4 and is fed to an absorber column 5. This gas is contacted with water introduced through conduit 7. Non-condensable gases exit overhead through conduit 6 while the majority of the product acrylonitrile, acetnonitrile, and other organics exit via an aqueous sidedraw at conduit 13. An aqueous bottoms stream containing some acrylonitrile, acetonitrile, and impurities exits through conduit 8 and is fed to an extractive distillation column 9. A portion of the aqueous bottoms stream is recycled through conduit 14 back to quench 2.

In a preferred embodiment, the bottom temperature of absorber 5 is maintained at a temperature of about 144 °F and the vapor exiting the pump-around section is maintained at approximately 100 °F. It is noted that the designs of extractive distillation columns are varied and frequently employ heat recovery devices and use recycle streams from point to point in the column or from other process units to optimize separation efficiency and/or economy. The exact design of this column and of the previously referenced quench and absorber columns are not critical to this invention and any commercially viable design can be utilized. In general, in extractive distillation columns, water is introduced through conduit 11 (usually located above the feed point of the bottoms stream from the absorber) to effect extractive distillation in the column which will normally contain 50-100 or more trays. To obtain optimum compositions for the preceding absorber column, the draw point of conduit 11 is, preferably, two to ten trays below the draw point of conduit 7. It is not essential that the draw point of conduit 11 be below that of conduit 7. Acrylonitrile and hydrogen cyanide are removed overhead through conduit 10. Preferably, acetonitrile is removed from the extractive distillation column through conduit 17. This is not essential but, otherwise, the overhead stream exiting the stripper through conduit 6 will contain significant amounts of acetonitrile.

Those skilled in the art will appreciate that all columns will be provided with necessary heat to effect their intended functions and that, for purposes of economy, much of such heat will be obtained from recycle streams used to supply processing liquid to the columns or to provide improved concentration/separation of various components. Such recycle and heat recovery techniques are conventional practice and, for simplicity, are not shown in the drawing or discussed in detail herein.

## Claims

1. In a process for purifying acrylonitrile in which an ammoxidation reactor effluent containing acrylonitrile and impurities is contacted in a quench system with an aqueous quench liquid; the improvement comprising condensing liquid from an overhead stream of the quench system and recycling the condensed liquid to the quench system prior to processing said liquid in a distillation column.

2. A process for purifying acrylonitrile as defined in Claim 1 wherein the overhead stream is condensed in a column with a pump-around loop.

3. A process for purifying acrylonitrile as defined in Claim 1 wherein the overhead stream is condensed in a partial condenser.

4. A process for purifying acrylonitrile as defined in Claim 1 wherein the condensation step is performed at a temperature of about 100-170°F.

5. A process for purifying acrylonitrile as defined in Claim 1 wherein 15-100% of the condensed liquid is recycled.

6. A process for purifying acrylonitrile as defined in Claim 1 wherein 25-50% of the condensed fluid is recycled.

7. A process for purifying acrylonitrile as defined in Claim 1 wherein about one-third of the condensed fluid is recycled.

8. In a process for purifying acrylonitrile in which an ammoxidation reactor effluent containing acrylonitrile and impurities is contacted in a quench system with an aqueous quench liquid; the improvement comprising condensing liquid from an overhead stream of the quench system in an absorber column and recycling a portion of the condensed liquid directly to the quench system.

9. A process for purifying acrylonitrile as defined in Claim 8 wherein the condensation step is performed at a temperature of about 100-170°F.

10. A process for purifying acrylonitrile as defined in Claim 8 wherein 15-100% of the condensed liquid is recycled.

11. A process for purifying acrylonitrile as defined in Claim 8 wherein about one-third of the condensed fluid is recycled.

12. In a process for purifying acrylonitrile in which an ammoxidation reactor effluent containing acrylonitrile and impurities is contacted in a quench system with an aqueous quench liquid; the improvement comprising condensing liquid from an overhead stream of the quench system in a partial condenser and recycling a portion of the condensed liquid directly to the quench system.

13. A process for purifying acrylonitrile as defined in Claim 12 wherein 15-100% of the condensed liquid is recycled.
